# EUROPÄISCHE PATENTSCHRIFT

(11) **EP 0 330 761 B1**
(45) Veröffentlichungstag und Bekanntmachung des Hinweises auf die Patenterteilung: **08.09.1993**
(21) Anmeldenummer: 88121877.0
(22) Anmeldetag: 30.12.1988
(51) Int. Cl.: A61M 1/10, A61M 1/14

(54) **Mess-/Anzeigeverfahren bei Flüssigkeitssystemen medizinischer Geräte und Vorrichtung zur Durchführung des Verfahrens**
Method for measuring and indicating in liquid systems of medical devices, and device for carrying out this method
Procédé de mesure et d'indication dans des systémes à liquide d'appareils médicaux et dispositif pour la mise en oeuvre du procédé

(30) Priorität: 27.02.1988 DE 3806248
(43) Veröffentlichungstag der Anmeldung: 06.09.1989
(73) Patentinhaber: Fresenius AG, 61350 Bad Homburg (DE)
(72) Erfinder: Polaschegg, Hans-Dietrich, Dr., D-6370 Oberursel 4 (DE)
(74) Vertreter: Fuchs Mehler Weiss

(56) Entgegenhaltungen:
- EP-A- 291 727
- EP-A- 0 172 117
- EP-A- 0 269 898
- WO-A-88/06466

## Beschreibung

Die Erfindung bezieht sich auf ein Verfahren zur Funktionsüberwachung (Ausfallerkennung) einer Druckmeßanordnung in einem Flüssigkeitssystem eines medizinischen Geräts, dessen Flüssigkeitsdruck von periodischen Druckschwankungen überlagert ist, wobei die Druckmeßanordnung bei korrekter Funktion ein dem momentanen Druckwert entsprechendes Meßsignal erzeugt sowie auf eine Vorrichtung zur Durchführung des Verfahrens mit einem Drucksensor in der Druckmeßanordnung eines Schutzsystems und einer Pumpenanordnung im Flüssigkeitssystem und einer dem Drucksensor nachgeschalteten Auswertanordnung für das Sensorausgangssignal.

Typische derartige medizinische Geräte sind Hämodialysegeräte und Infusionspumpen, anhand derer der Stand der Technik im folgenden gewürdigt wird, ohne daß die Erfindung auf diese Gerätetypen beschränkt wird.

Hämodialysegeräte verfügen in der Regel über ein Schutzsystem gegen Blutverlust in die Umgebung auf der Basis einer Drucküberwachung im extrakorporalen Schlauchsystem; tritt Blutverlust ein, sinkt der Druck ab und es wird ein Anzeigesignal erzeugt. Derartige Schutzsysteme sind z.B. in VDE 0750/Teil 206 beschrieben. Auch Infusionspumpen verfügen häufig über ein Schutzsystem, das eine Ruptur des Infusionsschlauches im Falle einer Okklusion verhindern soll.

In Hämodialysegeräten wird häufig auch die Überwachung des Dialysierflüssigkeitsdruckes oder des Transmembrandruckes als Schutzsystem gegen falsche Ultrafiltration herangezogen (VDE 0750/Teil 206).

Geräte der vorstehenden Art besitzen in der Regel Pumpen mit natürlichen/periodischen Förderratenschwankungen, insbesondere peristaltische Pumpen. Sie erzeugen daher pulsatile Strömungen, die sich aufgrund des Strömungswiderstandes als Druckschwankungen im Schlauchsystem bemerkbar machen.

Aus der EP 02 91 727 A2 ist eine Drucküberwachungsanordnung für eine medizinische Pumpenkassette bekannt geworden, bei der ein Drucksensor den Momentanwert des Flüssigkeitsdruckes im Flüssigkeitssystem der Pumpe einschließlich aller Druckschwankungen erfaßt und von den Drucksignalen Anzeige bzw. Alarmsignale abgeleitet werden.

Anhand des erstgenannten Schutzsystems gegen Blutverlust mit Überwachung des venösen Rücklaufdruckes im extrakorporalen Kreislauf sollen die Nachteile des Standes der Technik aufgezeigt werden.

Bei vg. Schutzsystem erfolgt die Wirkverbindung zwischen Drucksensor und dem extrakorporalen Kreislauf durch eine luftgefüllte Stichleitung. In diese Leitung ist in der Regel ein Hydrophobfilter oder eine bewegliche Membran eingefügt, die eine Kontamination des Drucksensors mit Blut verhindern soll, falls aufgrund einer Undichtigkeit Blut in die Stichleitung über eine bestimmtes Niveau eindringen sollte. Wird jedoch ein Hydrophobfilter benetzt oder die elastische Membran in eine Extremlage gebracht, erfolgt keine weitere Druckübertragung. Es wird quasi der vor dem Eintritt der Funktionsunfähigkeit angezeigte Druck "eingefroren". Ein weiterer Druckanstieg, der zur Ruptur des Schlauchsystems führen kann, wird nicht mehr registriert.

Analoges gilt für den Druckabfall.

Ein solcher Fehler kann durch den üblicherweise vorgeschriebenen Test des Schutzsystems vor Beginn der Dialyse nicht erkannt werden.

Der Stand der Technik vertraut daher mit Nachteil auf die Funktionsfähigkeit der Drucksensoren des Schutzsystems. Eher noch wird das elektrische Steuersystem aus Sicherheitsgründen verdoppelt (US-PS 42 98 938).

Der Erfindung liegt die Aufgabe zugrunde, bei den vorgenannten Drucküberwachungssystemen eine Funktionsüberwachung des Drucksensors und ihm unmittelbar zugeordneter Teile des Flüssigkeitssystems vorzusehen, um einen Ausfall oder eine Funktionsstörung erkennen zu können.

Zur Lösung dieser Aufgabe werden gemäß der Erfindung ausgehend von dem eingangs bezeichneten Verfahren die den periodischen Druckschwankungen entsprechenden Amplitudenschwankungen des Meßsignals erfaßt und als Kriterium für die korrekte Funktion der Druckmeßanordnung gewertet, wobei vorhandene Amplitudenschwankungen die korrekte Funktion, fehlende dagegen einen Ausfall der Druckmeßanordnung anzeigen.

Ausgehend von der eingangs bezeichneten Vorrichtung wird die Aufgabe erfindungsgemäß dadurch gelöst, daß die Auswerteanordnung neben einer Auswertestufe für die Überwachung der momentanen Druckwerte im Schutzsystem eine weitere Auswertestufe aufweist, die allein auf die periodischen Druckschwankungen anspricht und die an ihrem Ausgang ein Signal abgibt, wenn diese Druckschwankungen ausbleiben, das als Indikator für die Unwirksamkeit des Drucküberwachungssystems dient.

Das erfindungsgemäße Prinzip besteht somit darin, daß (kleine) periodische Druckschwankungen, die dem eigentlichen Druckwert überlagert sind, erfaßt und deren Fehlen im Auswertezweig als Fehlfunktion gewertet werden. Sollte daher das Drucksensorsystem unempfindlich werden, so wird nicht nur der statische Druck nicht mehr korrekt angezeigt, sondern auch die periodischen Druckschwankungen werden nicht mehr wiedergegeben. Überwacht man daher das Auftreten dieser Druckschwankungen, so kann das Ausbleiben derselben als Indikator für eine Unwirksamkeit des Schutzsystems herangezogen und das Gerät in einen sicheren Zustand überführt werden.

Sofern die natürlichen, durch die Bauart der Pumpe hervorgerufenen Druckschwankungen nicht ausreichen, um ein deutliches Anzeigesignal zu erzeugen, ist es nach einer Ausgestaltung der Erfindung zweckmäßig, die natürlichen Schwankungen zu verstärken und zusätzlich Förderratenschwankungen der Pumpe zu erzeugen, z.B. durch ungleichgemäße Rotationsgeschwindigkeiten bei peristaltischen Pumpen. Durch eine geeignete Steuerung der Rotationsgeschwindigkeit des Pumpenantriebes können so nahezu beliebige Pulsationen erzeugt werden, die den bauartspezifischen Pulsationen der Pumpe überlagert werden und diese entweder verstärken oder kompensieren.

Um zusätzlich zu einem Ausfall der Druckmeßanordnung auch noch eine Leitungsverstopfung im Flüssigkeitssystem erkennen zu können, ist nach einer Weiterbildung des erfindungsgemäßen Verfahrens vorgesehen, daß von der Phasenverschiebung zwischen einem von den Druckschwankungen am Ort ihrer Entstehung abgeleiteten Signal und einem Signal am Ort der Druckmessung ein Meßsignal abgeleitet wird, das ein Maß für den Strömungswiderstand im Flüssigkeitssystem zwischen beiden Orten ist.. Entsprechend weist bei der zugehörigen Vorrichtung die Auswerteanordnung eine weitere Auswertestufe auf, die die Phasendifferenz zwischen dem Sensorausgangssignal und dem periodischen Drucksignal am Ort der Pumpenanordnung erfaßt und mit einem Bezugssignal vergleicht.

Anhand von in der Zeichnung im Zusammenhang mit einem Hämodialysegerät dargestellten Ausführungs- und weiterer Anwendungsbeispielen wird die Erfindung näher erläutert. Dabei ergeben sich auch weitere Ausgestaltungen der Erfindung.

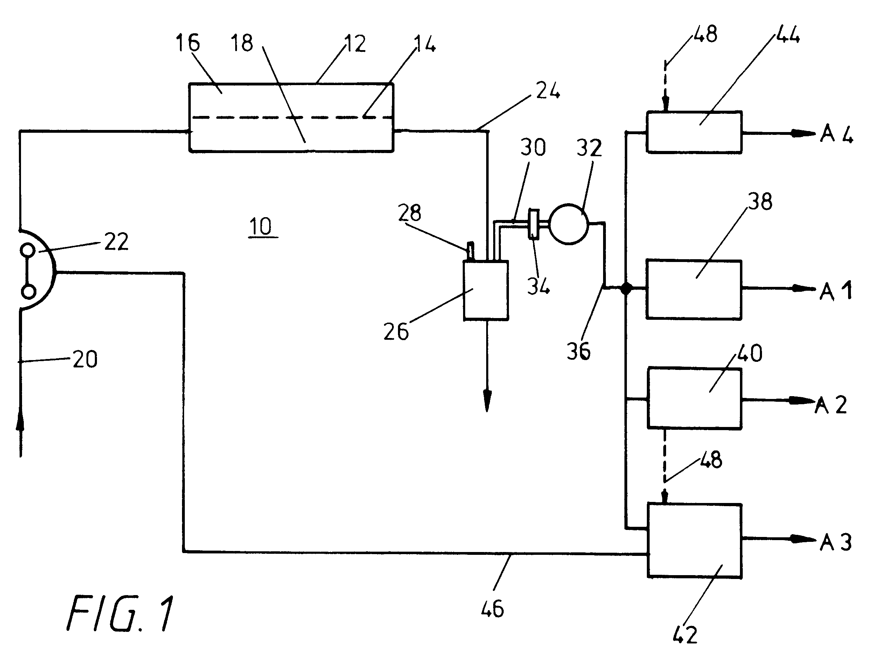

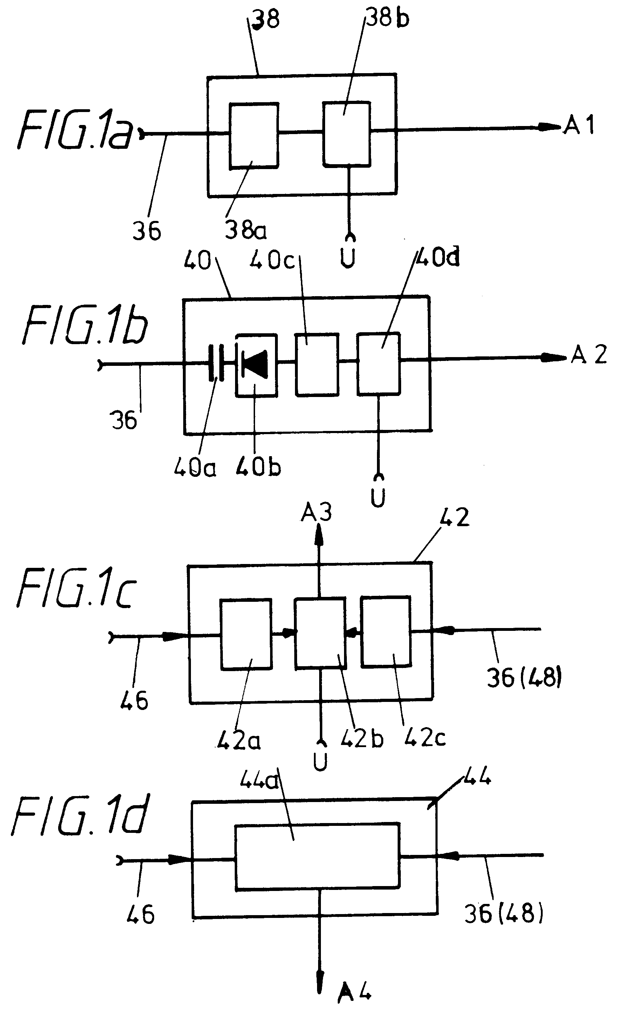

Dabei zeigt die Fig. 1 eine Gesamtübersicht und die Fig. 1a - 1d Ausgestaltungen der Auswertestufen.

Die Fig. 1 zeigt in schematischer Darstellung einen extrakorporalen Blutkreislauf 10 eines Hämodialysegerätes mit einem Dialysator 12, der durch eine Membran 14 in eine erste Kammer 16, in die üblicherweise ein nicht dargestellter Dialysierflüssigkeitsweg geschaltet ist, und in eine zweite Kammer 18 geteilt ist, in die der extrakorporale Kreislauf 10 gelegt ist. Dieser extrakorporale Kreislauf 10 besteht aus einer Zulaufleitung 20 und einer Rücklaufleitung 24.

In die Zulaufleitung 20 ist eine peristaltische Pumpe 22 geschaltet, mit der Blut aus den Arterien des Patienten gefördert wird.

In die Rücklaufleitung 24 ist eine venöse Tropfkammer 26 geschaltet, aus der ggf. mitgeführte Luft über einen Stutzen 28 abgeschieden wird. Des weiteren geht von der Tropfkammer 26 eine Stichleitung 30 ab, die mit einem Drucksensor 32 verbunden ist. Um den Drucksensor vor einer Blutbeaufschlagung zu schützen, ist in die Stichleitung 30 ein Sterilfilter 34 eingeschaltet, das aufgrund seiner mikroporösen, hydrophoben Eigenschaften einerseits die Stichleitung steril gegen die Umgebung abdichtet und andererseits kein Blut zum Drucksensor durchtreten läßt.

Der Drucksensor 32 ist über eine Leitung 36 mit einer elektrischen Auswerteschaltung 38 verbunden, die am Ausgang A1 den statischen venösen Rücklauf-Druck in üblicher Weise anzeigt und das Schutzsystem gegen Blutverlust in die Umgebung bildet. Zu diesem Zweck ist im einfachsten Fall, wie in Fig. 1a schematisch dargestellt, in der Auswerteschaltung 38 ein Schwellwertglied 38b vorgesehen, das mit dem Fachmann geläufigen elektronischen Schaltungen gebildet werden kann und das bei Über- bzw. Unterschreiten bestimmter Grenzwerte U des in der Stufe 38a geglätteten elektronischen Ausgangssignals des Drucksensors ein Alarmsignal abgibt.

Wie eingangs erläutert, kann dieses Schutzsystem unempfindlich und der vor Eintritt der Funktionsfähigkeit angezeigte Druck "eingefroren" werden. Ein weiterer Druckanstieg, der zur Ruptur des Schlauchsystems führen könnte, wird dann nicht mehr angezeigt.

Um ein Unwirksamwerden des Drucküberwachungssystems zu erkennen, ist eine zusätzliche zweite Auswertestufe 40 vorgesehen, die mittels bekannter elektronischer Schaltungen allein die periodischen Druckschwankungen im Schlauchsystem auswertet. Reichen die natürlichen Förderratenschwankungen der Blutpumpe 22 dabei nicht aus, um ein eindeutiges Meßsignal zu erhalten, werden mittels einer nicht dargestellten Pumpen-Steueranordnung künstliche Förderratenschwankungen erzeugt.

Sollte der Drucksensor 32 unempfindlich werden, so werden die periodischen Druckschwankungen nicht wiedergegeben. Die Auswertestufe 40 überwacht das Auftreten dieser Druckschwankungen und erzeugt bei deren Ausbleiben am Ausgang A2 ein Signal, das als Indikator für die Unwirksamkeit des Schutzsystems herangezogen werden kann und das Gerät in einen sicheren Zustand überführt.

In Fig. 1b ist ein einfaches Ausführungsbeispiel für die Auswertestufe 40 schematisch dargestellt.

Das Druck-Sensor-Ausgangssignal gelangt zunächst auf ein Entkoppelglied -hier in Form eines Kondensators 40a -, das dafür sorgt, daß nur Wechselspannungen durchgelassen werden. Fallen daher die periodischen Druckschwankungen durch einen Defekt weg, bleibt das Signal am Ausgang des Kondensators 40a aus.

Auf den Kondensator folgt ein Gleichrichter 40b sowie eine Glättungsstufe 40c, die aus dem Wechselspannungssignal eine Gleichspannung formen, die in einer Schwellwertstufe 40d mit einem Spannungsgrenzwert U verglichen wird. Diese Schwellwertstufe 40d gibt ein Signal ab, wenn der Grenzwert U unterschritten wird.

Die dargestellte Anordnung kann zusätzlich zur Überwachung der Veränderung des Strömungswiderstandes in der Schlauchleitung zwischen der Pumpe 22 und dem Drucksensor 32 herangezogen werden. Im Beispiel des Hämodialysegerätes liegt zwischen der Pumpe 22 und dem Drucksensor 32 ein Schlauchstück 20 von ca. 1 m Länge, der Dialysator 12 und ein weiteres Schlauchstück 24. Diese Schäuche besitzen eine gewisse Elastizität (oder Compliance), d.h. ihr Füllvolumen ändert sich mit dem Druckunterschied zwischen innen und außen. Diese Eigenschaft wirkt für Druckpulse wie eine Verzögerungsleitung, d.h. das Drucksignal am Ort des Drucksensors 32 ist gegenüber dem Signal, das am Ort des Entstehens der Druckpulse abgeleitet ist, also gegenüber der Rotation der peristaltischen Blutpumpe 22, phasenverschoben. Diese Phasenverschiebung hängt bei einer gegebenen, festen Anordnung vom Strömungswiderstand im Leitungssystem ab. Verändert sich dieser, so wird auch die Phasenverschiebung verändert.

Dieses Verhalten kann auch durch eine elektrisches Analogon erläutert werden. Die Pumpe 22 ist bei dieser Darstellung ein Frequenzgenerator, die Schlauchleitung eine RC-Kette und der Drucksensor 32 ein Oszillograph. In einer solchen Anordnung wird das mit vorgegebener Frewquenz alternierende Signal des Frequenz-Generators durch die RC-Kette verzögert. Beobachtet man also das Generatorsignal und das verzögerte Signal in einem Oszillographen, so wird eine Phasenverschiebung beobachtet, die sich bei Veränderung von R oder C ebenfalls verändert.

Zur Auswertung dieser Phasenverschiebung ist eine dritte Stufe 42 vorgesehen, die mit bekannten elektronischen Schaltungen einmal die Phasendifferenz zwischen dem periodisch schwankenden Drucksignal am Ausgang des Drucksensors 32 und dem entsprechenden Drucksignal am Ort der Pumpe 22 (bzw. einem von der Rotation der Pumpe 22 abgeleiteten Signal) erfaßt und daraus ein dem Strömungswiderstand proportionales Signal vergleicht, d.h. überwacht. Beim Anstieg des Strömungswiderstandes im Schlauchsystem zwischen Pumpe 22 und Sensor 32 erscheint daher am Ausgang A3 ein Signal, das eine Ruptur in diesem Teil des Dialysegerätes anzeigt.

In Fig. 1c ist ein einfaches Ausführungsbeispiel für die Auswertestufe 42 schematisch dargestellt. Am linken Eingang wird der Phasenerfassungsstufe 42a über die Leitung 46 das der Pumpe 22 zugeordnete und entsprechend schwankende Signal zugeführt. Am rechten Eingang wird der Phasenerfassungsstufe 42c über die Leitung 36 das periodisch schwankende Ausgangssignal des Drucksensors 32 zugeführt; dieses Signal kann auch - wie in Fig. 1 angedeutet - aus der Auswertestufe 40 (Fig. 1b) hinter dem Kondensator 40a bzw. dem Gleichrichter 40b über die Leitung 48 abgeleitet werden. Die Stufen 42a und c sind in Verbindung mit der Stufe 42b mit bekannten Mitteln so aufgebaut (z.B. unter Verwendung von Phasendetektoren, phasenempfindlichen Gleichrichtern etc.), daß sie abhängig von der Phasendifferenz der Signale an beiden Eingängen ein Gleichspannungssignal erzeugen, das mit der Bezugsspannung U verglichen wird. Beim Überschreiten bestimmter Phasendifferenzen erscheint am Ausgang A3 ein Alarmsignal.

Losgelöst von dem Gedanken der Funktionsüberwachung kann der Drucksensor 32 des extrakorporalen Kreislaufes nach Fig. 1 auch zur Registrierung des Pulses des Patienten herangezogen werden, der "in vivo" an den Kreislauf angeschlossen ist. Während der Behandlung wird der Druck im extrakorporalen Kreislauf sowohl durch den mit dem Pulsschlag schwankenden Blutdruck des Patienten als auch durch die Wirkung der Pumpe 22 bestimmt. Die periodischen Schwankungen überlagern sich. Der Pulsschlag kann nur detektiert werden, indem entweder die Blutpumpe 22 angehalten wird und die verbleibenden Druckschwankungen gemessen werden oder indem durch eine geeignete elektronische Auswertung die beiden Anteile frequenzmäßig separiert werden. Zur Ableitung eines entsprechenden Signals ist eine vierte Auswertestufe 44 vorgesehen, die je nach der gewählten Methode einen anderen Aufbau hat. Hält man die Blutpumpe kurzzeitig an, so werden die noch verbleibenden Druckschwankungen im extrakorporalen System ausschließlich durch den Puls des Patienten hervorgerufen und können in der Stufe 44 mit bekannten Mitteln entsprechend ausgewertet werden, an deren Ausgang A4 ein dem Pulsschlag entsprechendes Signal entsteht.

Im Fall der elektronischen Trennung beider Frequenzanteile enthält die Auswertestufe 44 eine sog. Frequenzweiche, die den von der Blutpumpe 22 hervorgerufenen Frequenzanteil, ausgedrückt durch die Pulsationsgeschwindigkeit der Blutpumpe 22 unterdrückt.

Dieser Fall ist in Fig. 1d schematisch dargestellt.

Die Auswertestufe enthält einen Frequenzanalysator 44a, der über den einen Eingang und die Leitung 46 mit dem gegenseitigen Signal und über den anderen Eingang und die Leitung 36 bzw. 48 (abgezweigt hinter dem Kondensator 40a der Stufe 1b) mit dem Drucksensorsignal beaufschlagt wird.

Zur Trennung der beiden Anteile kann auch ein Subtraktionsverfahren herangezogen werden, in dem vom Gesamtsignal das von der Drehzahl abgeleitete bekannte Pulsationssignal der Blutpumpe 22 in der Stufe 44 elektronisch abgezogen wird.

Die Auswertestufen sind in Fig. 1 nur funktionsmäßig getrennt dargestellt. Ihre elektronischen Schaltungen bilden einen Verbund, wobei einzelne Schaltungsteile, z.B. die Gleichspannungsversorgung, nur einfach vorhanden sind und diese Funktionen in mehreren Stufen ausüben.

Zur Anschaltung der einzelnen Komponenten der Geräte und der einzelnen Auswertestufen wird zweckmäßig ein Mikroprozessorsystem verwendet.

## Patentansprüche

1. Verfahren zur Funktionsüberwachung einer Druckmeßanordnung in einem Flüssigkeitssystem eines medizinischen Geräts, dessen Flüssigkeitsdruck von periodischen Druckschwankungen überlagert ist, wobei die Druckmeßanordnung bei korrekter Funktion ein dem momentanen Druckwert entsprechendes Meßsignal erzeugt, dadurch gekennzeichnet, daß die den periodischen Druckschwankungen entsprechenden Amplitudenschwankungen des Meßsignals erfaßt und als Kriterium für die korrekte Funktion der Druckanordnung gewertet werden, wobei vorhandene Amplitudenschwankungen die korrekte Funktion, fehlende dagegen einen Ausfall der Druckmeßanordnung anzeigen.

2. Verfahren nach Anspruch 1, dadurch gekennzeichnet, daß systeminhärenten periodischen Druckschwankungen zusätzlich erzeugte Druckschwankungen überlagert werden.

3. Verfahren nach Anspruch 1 oder 2, dadurch gekennzeichnet, daß von der Phasenverschiebung zwischen einem von den Druckschwankungen am Ort ihrer Entstehung abgeleiteten Signal und einem Signal am Ort der Druckmessung ein Meßsignal abgeleitet wird, das ein Maß für den Strömungswiderstand im Flüssigkeitssystem zwischen beiden Orten ist.

4. Vorrichtung, insbesondere zur Durchführung des Verfahrens nach Anspruch 1 oder 2 mit einem Drucksensor (32) in der Druckmeßanordnung eines Schutzsystems und einer Pumpenanordnung (22) im Flüssigkeitssystem und einer dem Drucksensor nachgeschalteten Auswerteanordnung für das Sensorausgangssignal, dadurch gekennzeichnet, daß die Auswerteanordnung neben einer Auswertestufe (38) für die Überwachung des momentanen Druckwerts im Schutzsystem eine weitere Auswertestufe (40) aufweist, die allein auf die periodischen Druckschwankungen anspricht und die an ihrem Ausgang ein Signal (12) abgibt, wenn diese Druckschwankungen ausbleiben, das als Indikator für die Unwirksamkeit des Drucküberwachungs- schutzsystems dient.

5. Vorrichtung nach Anspruch 4, insbesondere zur Durchführung des Verfahrens nach Anspruch 2, dadurch gekennzeichnet, daß eine Steueranordnung zur periodischen Veränderung der Förderrate der Pumpenanordnung (22) vorgesehen ist.

6. Vorrichtung nach Anspruch 5 mit einer peristaltischen Pumpe, dadurch gekennzeichnet, daß durch die Steueranordnung die Rotationsgeschwindigkeit der Pumpe (22) veränderbar ist.

7. Vorrichtung nach Anspruch 4, 5 oder 6, insbesondere zur Durchführung des Verfahrens nach Anspruch 3, dadurch gekennzeichnet, daß die Auswerteanordnung eine weitere Auswertestufe (42) aufweist, die die Phasendifferenz zwischen dem Sensorausgangssignal und dem periodischen Drucksignal am Ort der Pumpenanordnung (22) erfaßt und mit einem Bezugssignal (U) vergleicht.

8. Vorrichtung zur Messung des Pulsschlages eines Menschen, der "in vivo" an einen extrakorporalen Kreislauf angeschlossen ist, mit einer periodische Druckschwankungen erzeugenden Blutpumpe (22) und einer, auch Druckschwankungen erfassenden Druckmeßanordnung (32) und mit einer der Druckmeßanordnung nachgeschalteten Auswerteanordnung (38, 40) für die Drucksignale, dadurch gekennzeichnet, daß eine Steuerung vorgesehen ist, die so mit der Blutpumpe (22) verbunden ist, daß diese zum Meßzeitpunkt kurzzeitig anhaltbar ist, und daß die Auswerteanordnung eine weitere Auswertestufe (44) aufweist, die bei angehaltener Blutpumpe (22) direkt den Pulsschlag des Menschen erfaßt.

9. Vorrichtung zur Messung des Pulsschlages eines Menschen, der "in vivo" an einen extrakorporalen Kreislauf angeschlossen ist, mit einer periodische Druckschwankungen erzeugenden Blutpumpe (22) und einer, auch Druckschwankungen erfassenden Druckmeßanordnung (32) und mit einer der Druckmeßanordnung nachgeschalteten Auswerteanordnung (38, 40) für die Drucksignale, dadurch gekennzeichnet, daß die Auswerteanordnung eine weitere Auswertestufe (44) aufweist, die eine elektronische Weiche bzw. Substraktionsschaltung enthält, mittels der von dem Gesamt-Ausgangssignal an der Druckmeßanordnung elektronisch der von der Blutpumpe erzeugte Signalanteil unterdrückt bzw. kompensiert wird.

## Claims

1. Method for the functioning control of a pressure sensor in a fluid system of a medical apparatus whose fluid pressure is superimposed by periodical pressure fluctuations whereby the pressure measuring arrangement generates a measuring signal which at correct functioning corresponds to the instantanious pressure value, characterized in that the amplitude fluctuations of the measuring signal which correspond to the periodic pressure fluctuations are detected and evaluated as criterium for the correct functioning of the pressure arrangement, whereby existing amplitude fluctuations display the correct function, whereas missing ones display an outage of the measuring arrangement.

2. Method according to claim 1, characterized in that additionally generated pressure fluctuations are superimposed on system-inherent periodic pressure fluctuations.

3. Method according to claim 1 or 2, characterized in that from the phase displacement between a signal derived from the pressure fluctuations at the location of their formation and a signal at the location of the pressure measurement a measuring signal is derived which is a measure of the flow resistance between the two locations.

4. Apparatus, especially for carrying out the method according to claim 1 or 2, comprising a pressure sensor (32) in the pressure measuring arrangement of a protective system and a pump arrangement (22) in the fluid system and an evaluating arrangement following the pressure sensor for the sensor output signal, characterized in that the evaluating arrangement comprises apart from an evaluating stage (38) for the static pressure monitoring in the protective system a further evaluating stage (40) which responds solely to the periodic pressure fluctuations and which renders a signal at its outlet when said pressure fluctuations do not occur, which serves as indicator for the unefficiency of the pressure monitoring protective system.

5. Apparatus according to claim 4, especially for carrying out the method according to claim 2, characterized in that a control arrangement is provided for periodic variation of the delivery rate of the pump arrangement (22).

6. Apparatus according to claim 5 comprising a peristaltic pump, characterized in that by the control arrangement the rotational speed of the pump (22) is variable.

7. Apparatus according to claims 4, 5 or 6, especially for carrying out the method according to claim 3, characterized in that the evaluatiang arrangement comprises a further evaluating stage (42) which detects the phase difference between the sensor output signal and the periodic pressure signal at the location of the pump arrangement (22) and compares said difference with a reference signal (U).

8. Apparatus for measuring the pulse beat of a human being connected "in vivo" to an extracorporeal circuit, comprising a blood pump (22) generating periodic pressure fluctuations and a pressure measuring arrangement (32) detecting also pressure fluctuations, and comprising an evaluating arrangement (38, 40) for the pressure signals following the pressure measuring arrangement, characterized in that a control is provided being connected to the blood pump (22) such that same may be stopped momentarily at the time of measuring and that the evaluation arrangement comprises a further evaluating stage (44) which detects the pulse beat of the human being directly with the blood pump (22) stopped.

9. Apparatus for measuring the pulse beat of a human being connected "in vivo" to an extracorporeal circuit, comprising a blood pump (22) generating periodic pressure fluctuations and a pressure measuring arrangement (32) detecting also pressure fluctuations, and comprising an evaluating arrangement (38, 40) for the pressure signals following the pressure measuring arrangement, characterized in that the evaluating arrangement comprises a further evaluating stage (44) which comprises an electronic shunt or subtraction circuit, respectively, by means of which the signal portion of the total output signal at the pressure measuring arrangement generated by the blood pump is electronically suppressed or compensated, respectively.

## Revendications

1. Procédé pour contrôler le fonctionnement d'un dispositif manométrique dans un système de liquide d'un appareil médical, à la pression du liquide duquel sont superposées des variations périodiques de pression, le dispositif manométrique délivrant un signal de mesure correspondant à la valeur de pression instantanée, dans le cas d'un fonctionnement correct, caractérisé en ce que les variations d'amplitude du signal de mesure, qui correspondent aux variations périodiques de pression, sont détectées et sont évaluées en tant que critère pour le fonctionnement correct du dispositif manométrique, l'existence de variations d'amplitude indiquant un fonctionnement correct, tandis que l'absence de variations d'amplitude indique une défaillance du dispositif manométrique.

2. Procédé selon la revendication 1, caractérisé en ce qu'en outre des variations de pression produites de façon supplémentaire sont superposées aux variations périodiques de pression, qui sont propres au système.

3. Procédé selon la revendication 1 ou 2, caractérisé en ce qu'à partir du déphasage entre un signal dérivé des variations de pression à l'emplacement d'apparition de ces dernières et un signal présent à l'emplacement de mesure de la pression est dérivé un signal de mesure qui est une mesure de la résistance d'écoulement dans le système de liquide entre deux emplacements.

4. Dispositif, notamment pour la mise en oeuvre du procédé selon la revendication 1 ou 2, comportant un capteur de pression (32) situé dans le dispositif manométrique d'un système de protection et d'un dispositif à pompe (22) dans le système de liquide, et un dispositif d'évaluation branché en aval du capteur de pression et servant à évaluer le signal de sortie du capteur, caractérisé en ce que le dispositif d'évaluation comporte, en dehors d'un étage d'évaluation (38) servant à contrôler la valeur de pression instantanée dans le système de protection, un autre étage d'évaluation (40), qui répond uniquement aux variations périodiques de pression et délivre un signal (12) à sa sortie lorsque ces variations de pression sont absentes, ce signal étant utilisé comme indicateur de l'inefficacité du système de contrôle de pression et de protection.

5. Dispositif selon la revendication 4, notamment pour la mise en oeuvre du procédé selon la revendication 2, caractérisé en ce qu'il est prévu un dispositif de commande pour modifier périodiquement le débit de refoulement du dispositif à pompe (22).

6. Dispositif selon la revendication 5 comportant une pompe péristaltique, caractérisé en ce que la vitesse de rotation de la pompe (22) est modifiable au moyen du dispositif de commande.

7. Dispositif selon la revendication 4, 5 ou 6, notamment pour la mise en oeuvre du procédé selon la revendication 3, caractérisé en ce que le dispositif d'évaluation comporte un autre étage d'évaluation (42), qui détecte le déphasage entre le signal de sortie du capteur et le signal de pression périodique à l'emplacement du dispositif à pompe (22) et le compare à un signal de référence (U).

8. Dispositif pour la mesure du pouls chez la personne, qui est raccordé "in vivo" à un circuit extracorporel, comportant une pompe à sang (22) produisant des variations périodiques de pression, un dispositif manométrique (32) détectant également des variations de pression, et comportant un dispositif d'évaluation (38,40) branché en aval du dispositif manométrique et servant à évaluer les signaux de pression, caractérisé en ce qu'il est prévu une commande qui est raccordée à la pompe à sang (22) de telle sorte que cette dernière peut être arrêtée pendant un bref intervalle de temps à l'instant de mesure et que le dispositif d'évaluation comporte un autre étage d'évaluation (44) qui détecte directement le pouls de la personne lorsque la pompe à sang (22) est arrêtée.

9. Dispositif pour la mesure du pouls chez la personne, qui est raccordé "in vivo" à un circuit extracorporel, comportant une pompe à sang (22) produisant des variations périodiques de pression, un dispositif manométrique (32) détectant également des variations de pression, et comportant un dispositif d'évaluation (38,40) branché en aval du dispositif manométrique et servant à évaluer les signaux de pression, caractérisé en ce que le dispositif d'évaluation comporte un autre étage d'évaluation (44) qui contient un aiguillage électronique ou un circuit soustracteur, au moyen duquel la composante du signal, qui est produite par la pompe à sang, est supprimée électroniquement du signal global de sortie au niveau du dispositif manométrique ou est compensée.
